# EUROPEAN PATENT APPLICATION

(11) **EP 4 653 526 A1**
(43) Date of publication of application: **26.11.2025**
(21) Application number: 24799112.8
(22) Date of filing: 10.09.2024
(51) Int. Cl.: C12N 9/26, C12N 15/85

(54) **METHOD FOR PRODUCING INTACT FORM OF HYALURONIDASE**

(30) Priority: 09.04.2024 KR 20240047958
(71) Applicant: Huonslab Co., Ltd., Seongnam-si, Gyeonggi-do 13201 (KR)
(72) Inventor: LIM, Chaeyoung, Gangnam-gu, Seoul 06155 (KR); KIM, Donghwan, Giheung-gu, Yongin-si, Gyeonggi-do 16931 (KR); KIM, Kyungsik, Michuhol-gu, Incheon 22230 (KR); BYYUNG, Juyoung, Suji-gu, Yongin-si, Gyeonggi-do 16822 (KR); OH, Cheolwoo, Jungwon-gu, Seongnam-si, Gyeonggi-do 13356 (KR); PARK, Boyoung, Giheung-gu, Yongin-si, Gyeonggi-do 17013 (KR)
(74) Representative: J A Kemp LLP
(86) International application number: PCT/KR2024/013731
(87) International publication number: WO 2025/216369

(57) **Abstract**

The present invention relates to a method for producing original form hyaluronidase, and more specifically, to a method for producing original form hyaluronidase PH20 through the control of the culture temperature or medium ingredients of host cells expressing natural human recombinant hyaluronidase PH20.

In the present invention, through the control of the culture temperature, medium type, added feed medium ingredients, dissolved oxygen concentration, glucose concentration, pH, and the like of host cells expressing natural human recombinant hyaluronidase (HAdase) PH20, original form HAdase PH20, not a truncated form of HAdase PH20, was produced with high purity. The original form HAdase PH20 produced by the method of the present invention has high specific activity, and thus can be effectively applied to various fields.

## Description

### [Technical Field]

The present invention relates to a method for producing original form hyaluronidase, and more specifically, to a method for producing original form hyaluronidase PH20 through the control of the culture temperature or medium components of host cells expressing natural human recombinant hyaluronidase PH20.

### [Background Art]

Hyaluronidase (HAdase) is a general term for enzymes that degrade hyaluronic acid (HA). HAdase is classified into mammalian type HAdase (EC 3.2.1.35, hyaluronoglucosaminidase), leech type HAdase (EC 3.2.1.36, hyaluronoglucuronidase), and bacterial type HAdase (EC 4.2.2.1, hyaluronate lyase) depending on the mechanism by which it hydrolyzes hyaluronic acid.

Mammalian HAdase is present in the testes, skin, liver, and placental fluid of the human body, and has the characteristic of hydrolyzing the β-1,4 glycosidic bond between glucuronic acid and glucosamine, which are components of HA, to produce tetrasaccharides, or hydrolyzing chondroitin, chondroitin-4-sulfate, and chondroitin-6-sulfate, which are components of synovial fluid and cartilage in the joints of the human body. In particular, HAdase in the testes (PH-20) is an important enzyme that is attached to the glycosylphosphatidylinositol (GPI) anchor site of the acrosome of a spermatozoon and decomposes the thick outer wall layer of the egg to cause fertilization.

The extensive use of HAdase has been comprehensively reviewed since the 1950s, and the first use was subcutaneous injection of fluids. Other uses include infiltration and blocking anesthesia to increase the diffusion of local anesthetics and steroids in orthopedic, ophthalmological, plastic, dental, oral, gynecological, and otolaryngological surgeries, dispersing body fluids such as hematomas, preventing peritoneal adhesions, preventing kidney stone formation, and treating infertility.

Currently, commercially available HAdases are extracted from ovine or bovine testicles. Examples of these include Vitrase (ISTA Pharmaceuticals, ovine source) and Amphadase (Amphastar Pharmaceuticals, bovine source). These unprocessed HAdases are dissolved at an appropriate concentration, filled into vials, and then lyophilized to produce commercialized products. Commercialized animal-derived HAdases contain foreign proteins, which may cause allergic reactions, and the decrease in their stability over time decreases physiological activity, leaving many problems on application in various fields.

To overcome these problems, research on recombinant HAdase has been conducted. Recombinant proteins may be expressed in various types of cells, such as *E. coli,* yeast, insect cells, and animal cells. In particular, HAdase undergoes glycosylation that occurs during the post-translational modification process of proteins, which affects its activity. This is because glycans may affect the antigenicity, structural folding, availability, and stability of glycoproteins. From this perspective, in the case of yeast or insect cells that undergo glycosylation, since the patterns of the post-translational modification process of proteins are different from those of mammals, animal cells are suitable among various types of expression cells, and among animal cells, Chinese hamster ovary (CHO) cells, whose safety has been ensured, are the most suitable.

The first recombinant HAdase for PH20 is being sold under the trade name Hylenex by Halozyme Therapeutics and is under development for various applications such as subcutaneous injection, vitrectomies, and ophthalmic disorders. However, HAdase still has low yield or stability and the supply is low compared to demand, so HAdase with improved yield or stability is required (Korean Patent Publication No. 10-2023-0168902; Korean Patent Registration No. 10-2528707).

In order to increase the production yield of recombinant HAdase in conventional technology, a production method in which the content of N-glycan of recombinant HAdase is changed by controlling glucose concentration or culture temperature is known (Korean Patent Publication No. 10-2022-0018943), but this method has a problem in that HAdase is produced in a truncated form rather than an original form.

### [Disclosure]

### [Technical Problem]

Accordingly, in the present invention, efforts were made to increase the productivity of original form hyaluronidase (HAdase) rather than a truncated form of HAdase, and as a result, it was confirmed that the production of original form HAdase PH20 was possible by controlling the culture temperature, medium components, and added feed medium components of host cells expressing natural human recombinant HAdase PH20, thereby completing the present invention.

Therefore, one object of the present invention is to provide a method for producing original form HAdase PH20 by controlling the culture temperature or medium components of host cells expressing natural human recombinant HAdase PH20.

### [Technical Solution]

To achieve the above-described object, the present invention provides a method for producing original form hyaluronidase (HAdase) PH20, including:
(1) culturing a host cell expressing natural human recombinant HAdase PH20 at a culture temperature of 33 to 35 °C; and
(2) culturing for 7 to 11 days while maintaining the culture temperature at 31 to 33 °C,
wherein the host cell in Step (1) and/or Step (2) is cultured in a CD OptiCHO culture medium to which Cell Boost 6 feed medium is added, and
the produced HAdase PH20 is present in an original form having a size of 50 to 75 kDa and not in a truncated form.

In one preferred embodiment of the present invention, the original form HAdase PH20 may have a size of 55 to 65 kDa.

In another preferred embodiment of the present invention, the activity of the HAdase PH20 produced by the method in a culture solution may be 4,000 units/ml or higher.

In still another preferred embodiment of the present invention, the Cell Boost 6 feed medium may be contained in the culture medium of the host cells in Step (1) and/or Step (2) at a concentration of 1% (v/v) to 20% (v/v), preferably at a concentration of 1% (v/v) to 15% (v/v).

In yet another preferred embodiment of the present invention, L-glutamine may further be added to the culture medium of the host cells in Step (1) and/or Step (2) and cultured.

In yet another preferred embodiment of the present invention, no component selected from the group consisting of peptone, plant-derived hydrolysate, yeast extract, and yeast hydrolysate (yeastolate) may be added to the culture medium of the host cells in Step (1) and/or Step (2).

In yet another preferred embodiment of the present invention, the culture in Step (2) may be performed by one or more methods selected from the group consisting of: (a) culturing in which the dissolved oxygen content of a culture solution is maintained at 30% to 79%; (b) culturing in which the residual glucose concentration in the medium is maintained at 1.11 to 5.95 g/l during the culture period; and (c) culturing in which the pH of the culture solution is maintained at 7.0 to 7.4.

In yet another preferred embodiment of the present invention, the culture of the host cells in Step (1) and/or Step (2) may be performed by one or more methods selected from the group consisting of batch culture, repeated batch culture, fed-batch culture, repeated fed-batch culture, continuous culture, and perfusion culture.

In yet another preferred embodiment of the present invention, the HAdase PH20 produced by the method may optionally have some of the amino acid residues at an N-terminus and/or C-terminus deleted.

In yet another preferred embodiment of the present invention, the host cell may be an animal cell, yeast, *Actinomycetes,* or an insect cell.

In yet another preferred embodiment of the present invention, the production method may further include (3) separating and purifying the produced HAdase PH20.

In yet another preferred embodiment of the present invention, the separating and purifying in Step (3) may be to purify HAdase PH20 with high purity by utilizing affinity binding, ionic binding characteristics, and/or hydrophobic interaction characteristics with PH20.

In yet another preferred embodiment of the present invention, the separating and purifying may be performed using affinity chromatography, ion exchange-mixed mode chromatography, and hydrophobic interaction chromatography.

In addition, the present invention provides original form hyaluronidase PH20 having a size of 50 to 75 kDa produced by the method for producing original form HAdase PH20.

### [Advantageous Effects]

In the present invention, through control of the culture temperature, medium type, added feed medium components, dissolved oxygen concentration, glucose concentration, pH, and the like of host cells expressing natural human recombinant HAdase PH20, original form HAdase PH20, not a truncated form of HAdase PH20, was produced with high purity. The original form HAdase PH20 produced by the method of the present invention has high specific activity, and thus can be effectively applied to various fields.

### [Description of Drawings]

FIG. 1 shows Western blot data confirming the expression of a truncated form (molecular weight standard between 37 and 49 kDa) or an original form (molecular weight standard between 50 and 75 kDa) of natural human recombinant hyaluronidase (HAdase) PH20 according to the main culture conditions of the present invention, such as culture temperature and temperature change days.
FIG. 1A shows the analytical results according to the culture temperature (34, 37 °C → 32, 34, 37 °C), temperature change day (days 3, 5, 7, 9), and additional feed (Feed C+, GlycanTune C+) when the basic batch was performed with HyCell medium.
FIG. 1B shows the analytical results according to the culture temperature (34, 37 °C → 32, 34, 37 °C), temperature change day (days 3, 5, 7, 9), and additional feed (Feed C+, Cell Boost 6) when the basic batch was performed with CD OptiCHO medium.
FIG. 2 shows the Western blot data confirming the expression of a truncated form or an original form of natural human recombinant HAdase PH20 according to the type of culture additives and the feed content.
FIG. 2A shows the analytical results according to the Cell Boost 6 content (3.75, 5, 7.5, 15 mM) and addition day (days 0, 3, 6, 9) in the presence of the L-glutamine additive.
FIG. 2B shows the analysis results according to the Cell Boost 6 content (3.75, 5, 7.5, 15 mM) and addition day (days 0, 3, 6, 9) in the presence of the GlutaMAX (4 mM) additive.
FIG. 3 shows the Western blot data confirming the expression of a truncated form or an original form of the natural human recombinant HAdase PH20 when peptone was added as a culture additive according to the addition content (3, 9%) and the type of peptone (yeast extract, Select Phytone, TC Yeastolate, Cotton 200).
FIG. 4 shows the Western blot data confirming the expression of a truncated form or an original form of the natural human recombinant HAdase PH20 according to the basic culture conditions and culture days. The basic culture conditions were [CD OptiCHO + Cell Boost 6 (15%)] and [CD OptiCHO + Cell Boost 6 (15%) + TC Yeastolate (9%)], and the culture days were set to 12, 14, 16, 18, and 20 days.
FIG. 5 shows the Western blot data confirming the expression of a truncated form or an original form of the natural human recombinant HAdase PH20 according to the (A) culture days, (B) culture agitation speed, and (C) medium formulation of the final culture conditions.
FIG. 6 shows the first purification chromatogram by ion exchange-mixed mode chromatography according to the purification flow speed of the natural human recombinant HAdase PH20.
FIG. 7 shows the second purification chromatogram by affinity chromatography according to the purification flow speed of the natural human recombinant HAdase PH20.
FIG. 8 shows the third purification chromatogram by ion exchange-mixed mode chromatography according to the purification flow speed of the natural human recombinant HAdase PH20.
FIG. 9 shows the fourth purification chromatogram by mixed mode chromatography according to the purification flow speed of the natural human recombinant HAdase PH20.
FIG. 10 shows the fifth purification chromatogram by hydrophobic interaction chromatography according to the purification flow speed of the natural human recombinant HAdase PH20.
FIG. 11 shows the results of Western blot analysis of the natural human recombinant HAdase PH20 product obtained through the final purification process.
FIG. 12 shows the results of sodium dodecyl sulfate-polyacrylamide gel electrophoresis (SDS-PAGE) analysis of the natural human recombinant HAdase PH20 product obtained through the final purification process.
FIG. 13 shows the results of size exclusion chromatography analysis of the natural human recombinant HAdase PH20 product obtained through the final purification process.

### [Modes of the Invention]

Hereinafter, the present invention will be described in detail.

One aspect of the present invention relates to a method for producing original form hyaluronidase (HAdase) PH20, including:
(1) culturing a host cell expressing natural human recombinant HAdase PH20 at a culture temperature of 33 to 35 °C; and
(2) culturing for 7 to 11 days while maintaining the culture temperature at 31 to 33 °C,
wherein the host cell in Step (1) and/or Step (2) is cultured in a CD OptiCHO culture medium to which Cell Boost 6 feed medium is added, and
the produced HAdase PH20 is present in an original form having a size of 50 to 75 kDa and not in a truncated form.

In the present invention, the culture in Step (1) may be performed so that the integral viable cell density reaches 0.3 x 10⁶ to 13.1 x 10⁶ cells x day/ml.

In the present invention, the original form HAdase PH20 may have a size of 50 to 75 kDa, preferably 58 to 65 kDa, more specifically 55 to 63 kDa (about 60 kDa). The production of the original form HAdase PH20 is 90% or higher, preferably 95% or higher, more preferably 97% or higher, and HAdase PH20 in a truncated form (37 to 49 kDa) is substantially not observed.

In the present invention, the CD OptiCHO culture medium (Gibco, USA) include additives (glucose, sodium pyruvate, sodium bicarbonate) and inorganic salts (magnesium, sodium phosphate), and Cell Boost 6 (HyClone, USA) includes lipids, amino acids, vitamins, growth factors, and the like. The Cell Boost 6 feed medium may be added to the CD OptiCHO culture medium at a concentration of 1% (v/v) to 20% (v/v), preferably 1% (v/v) to 15% (v/v) for culture.

The "feed medium" used herein is a medium composed of specific nutrients or multiple nutrients such as amino acids, vitamins, salts, trace elements, lipids, and glucose, and may be a concentrated product of a basic medium, and the feed medium may be produced by varying the components and concentrations thereof depending on the cells to be cultured. In the present invention, Cell Boost Series supplement medium (HyClone, USA) was preferably used as a feed medium.

In the present invention, L-glutamine may further be added to the culture medium of the host cells in Step (1) and/or Step (2) and cultured, and glutamine may be added to the CD OptiCHO medium at a concentration of 2 to 6 mM, more preferably at a concentration of 3 to 5 mM.

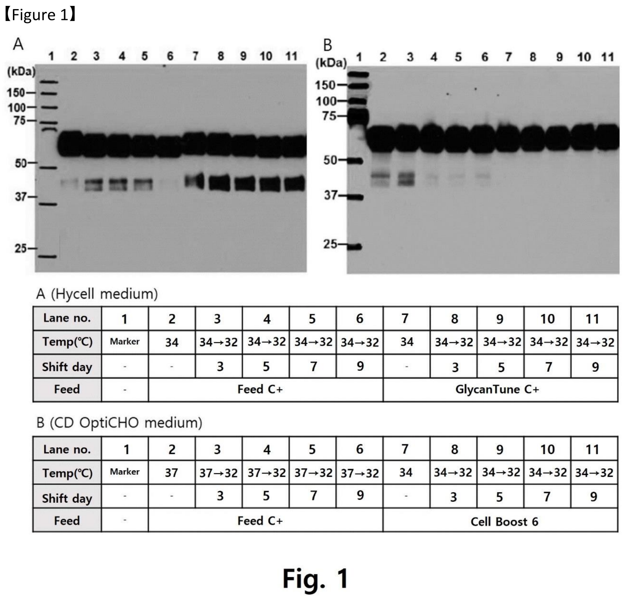

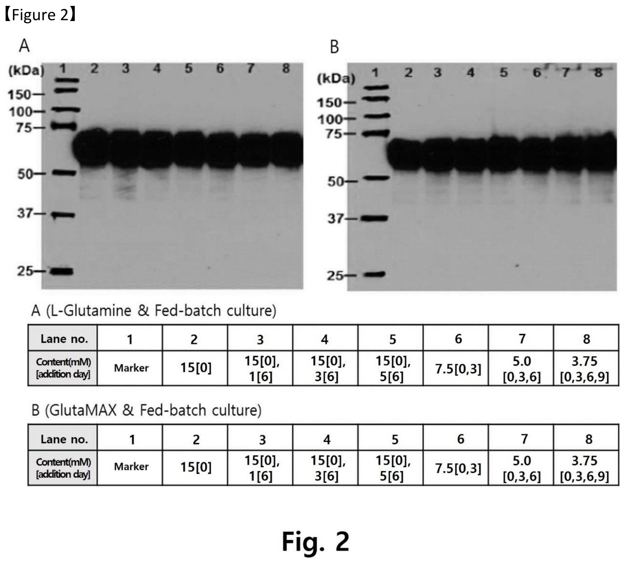

In one specific embodiment of the present invention, in order to produce original form HAdase PH20, an attempt was made to establish optimal conditions according to the type of commercial medium and culture temperature. As a result, when the initial culture temperature was set to 34 °C under CD OptiCHO medium conditions and Cell Boost 6 was added as an additional feed, a truncated form of PH20 was hardly observed regardless of the culture temperature change day (FIG. 1). In addition, no significant difference in the degree of expression of the truncated form of PH20 was observed according to the addition of L-glutamine (FIG. 2).

In the present invention, no component selected from the group consisting of peptone, plant-derived hydrolysate, yeast extract, and yeast hydrolysate (yeastolate) may be added to the culture medium of the host cells in Step (1) and/or Step (2).

The plant-derived hydrolysates refer to products extracted from garden peas, cottonseed, or wheat gluten, soybeans, and the like, and commercially available plant-derived hydrolysates include, for example, Hy-Pea^{™} 7404, UltraPep^{™} Cotton, HyPep^{™} 7504, HyPep^{™} (all from Kerry, USA), Cotton 100, Cotton 200, Phytone^{™} Soy 100 (all from Gibco, USA).

The peptones, plant-derived hydrolysates, yeast extracts or yeast hydrolysates are rich in amino acids, peptides, vitamins, carbohydrates, nucleotides, minerals, and other components, and are therefore widely used as cell culture supplements or additives. However, in the present invention, it was confirmed that the expression of a truncated form of HAdase PH20 was increased by them, so they are not suitable for the production of original form HAdase PH20.

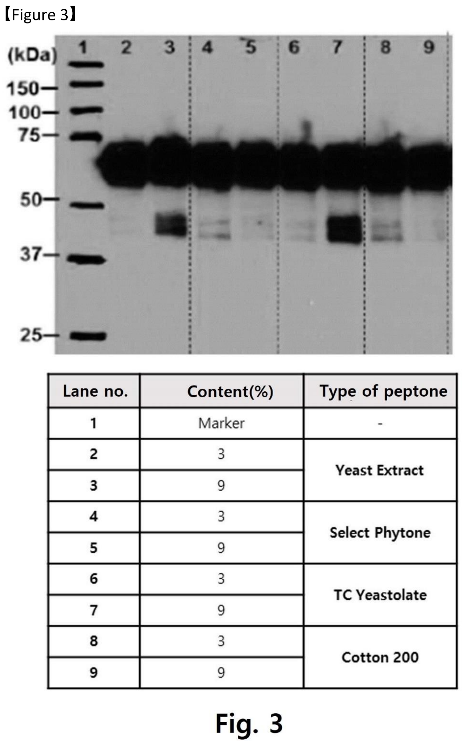

In one specific embodiment of the present invention, it was confirmed that when peptones or TC yeastolate of various origins were added as additives, a truncated form of HAdase PH20 was expressed under all culture conditions (FIG. 3).

In the present invention, the culture in Step (2) may be performed by one or more methods selected from the group consisting of: (a) culturing in which the dissolved oxygen content of a culture solution is maintained at 30% to 79%; (b) culturing in which the residual glucose concentration in the medium is maintained at 1.11 to 5.95 g/l during the culture period; and (c) culturing in which the pH of the culture solution is maintained at 7.0 to 7.4.

To produce original form HAdase PH20, the culture may be performed while maintaining the dissolved oxygen content of a culture solution at 30% to 79%, preferably at 30% to 50%, more preferably at 30% to 35%.

The culture may be performed while maintaining the residual glucose concentration in a culture solution at 1.11 to 5.95 g/l, preferably 3.5 g/l to 5.95 g/l, but is not limited thereto.

The fact that the culture is performed while maintaining the dissolved oxygen content, glucose concentration, or pH of a culture solution means that the dissolved oxygen content, glucose concentration, or pH in a culture solution are measured at intervals of every 1 to 36 hours, preferably every 3 to 30 hours, and more preferably every 6 to 24 hours, or on a real-time basis, during the culture period, and when the concentration is lower than a set reference concentration, the concentration is adjusted to reach the reference concentration.

In the present invention, the culture of the host cells in Step (1) and/or Step (2) may be performed by one or more methods selected from the group consisting of batch culture, repeated batch culture, fed-batch culture, repeated fed-batch culture, continuous culture, and perfusion culture.

The "HAdase PH20" or "PH20" according to the present invention may be interpreted to mean both natural PH20 and a mature form thereof, and the HAdase PH20 produced by the method may optionally have some of the amino acid residues at an N-terminus and/or C-terminus deleted, but is not limited thereto.

The human-derived "HAdase PH20" according to the present invention may be represented by the amino acid sequence of SEQ ID NO: 1, but a human-derived HAdase PH20 known in the art may be used.

In the present invention, the host cell may be an animal cell, yeast, *Actinomycetes,* or an insect cell, but is not limited thereto.

The animal cells are preferably mammalian cells. More preferably, commonly used animal culture cells such as CHO cells, human embryonic kidney (HEK) cells, CV-1 in origin and carrying SV40 (COS) cells, 3T3 cells, myeloma cells, baby hamster kidney (BHK) cells, HeLa cells, and Vero cells are used, and CHO cells are particularly preferred when the purpose is mass expression. In addition, in order to produce a desired protein, cells suitable for introducing a desired gene, such as dhfr-CHO cells (Proc Natl Acad Sci USA, 77:4216-4220, 1980), which are dihydrofolate reductase (DHFR) gene deficient CHO cells, or CHO K-1 cells (Proc Natl Acad Sci USA, 60:1275, 1968), are particularly preferred. As the above CHO cells, the DG44 strain, DXB-11 strain, K-1 strain, or CHO-S strain is particularly preferable, and introduction of the vector into the host cell may be carried out by a method such as calcium phosphate method, diethylaminoethyl (DEAE)-dextran method, electroporation method, or lipofection.

Examples of yeast include *Saccharomyces* sp., *Hansenula* sp., *Kluyveromyces,* and *Pichia* sp., and examples of *Actinomycetes* include *Streptomyces,* but are not limited thereto.

In the present invention, the production method may further include (3) separating and purifying the produced HAdase PH20.

In the present invention, a truncated form of HAdase PH20 may be completely removed through the separating and purifying in Step (3).

In the present invention, the separating and purifying in Step (3) may be to purify HAdase PH20 with high purity by utilizing affinity binding, ionic binding characteristics, and/or hydrophobic interaction characteristics with HAdase PH20.

In the present invention, the separating and purifying of PH20 may be performed using affinity chromatography, ion exchange-mixed mode chromatography, and hydrophobic interaction chromatography.

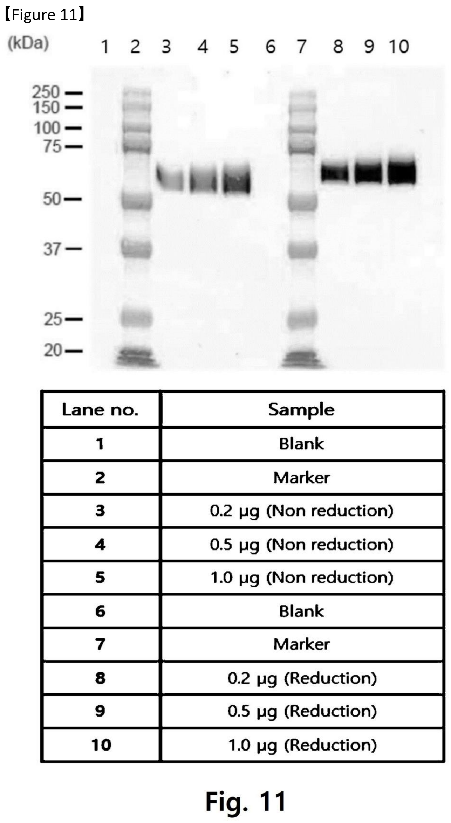

In one specific embodiment of the present invention, the produced HAdase PH20 was purified through step 1: ion exchange-mixed mode chromatography -> step 2: affinity chromatography -> step 3: virus inactivation -> step 4: ion exchange-mixed mode chromatography -> step 5: mixed mode chromatography -> step 6: hydrophobic interaction chromatography -> step 7: ultrafiltration (UF) /diafiltration (DF) and virus filtration, and as a result of analyzing the purified HAdase PH20, it was confirmed that original form HAdase PH20, not a truncated form, was purified (FIGS. 11 to 13).

Another aspect of the present invention relates to original form HAdase PH20 having a size of 50 to 75 kDa produced by the method for producing original form HAdase PH20.

In the present invention, original form HAdase PH20, not a truncated form, was produced with high purity. The original form HAdase PH20 produced by the method of the present invention has high specific activity, and thus can be effectively applied to various fields.

Hereinafter, the present invention will be described in more detail through examples.

These examples are intended only to illustrate the present invention, and it will be obvious to those skilled in the art that the scope of the present invention is not to be construed as being limited by these examples.

### Example 1: Exploration of commercial media, additives, and culture temperature

The optimal commercial medium and culture temperature were explored using host cells (DG44 CHO cells, SBA002) expressing native human recombinant HAdase PH20 (SEQ ID NO: 1).

As the commercial medium, CDM4CHO (GE Healthcare), HyCell CHO (GE Healthcare), EX-CELL CD CHO (Merck), PowerCHO-2 CD (Lonza), CD OptiCHO (Thermo Fisher Scientific), and CD CHO 022 (JS Biosciences) were used, and the host cells were cultured at a culture temperature of 34 to 37 °C.

As the additives, Feed A+ (Thermo Fisher Scientific), Feed B+ (Thermo Fisher Scientific), Feed C+ (Thermo Fisher Scientific), GlycanTune C+ (Thermo Fisher Scientific), Cell Boost 6 (HyClone), and CD Feed 002 (Irvine Scientific) were used.

**[Table 1]**

| Cell viability according to medium type, additives, and culture temperature | | | | | |
|---|---|---|---|---|---|
| No. | Medium | Additive | Culture temperature (°C) | Culture days | Cell concentration (x 10⁶ cell/ml) |
| 1 | CDM4CHO | Feed A+ | 34 | 14 | 14.58 |
| 2 | CDM4CHO | Feed B+ | 34 | 13 | 12.43 |
| 3 | CDM4CHO | Feed C+ | 34 | 16 | 9.63 |
| 4 | CDM4CHO | GlycanTune C+ | 34 | 16 | 8.21 |
| 5 | HyCell CHO | Feed A+ | 34 | 16 | 29.76 |
| 6 | HyCell CHO | Feed B+ | 34 | 16 | 27.72 |
| 7 | HyCell CHO | Feed C+ | 34 | 16 | 25.08 |
| 8 | HyCell CHO | GlycanTune C+ | 34 | 16 | 22.91 |
| 9 | EX-CELL CD CHO | Feed A+ | 34 | 16 | 17.16 |
| 10 | EX-CELL CD CHO | Feed B+ | 34 | 15 | 16.66 |
| 11 | EX-CELL CD CHO | Feed C+ | 34 | 16 | 11.37 |
| 12 | EX-CELL CD CHO | GlycanTune C+ | 34 | 16 | 10.74 |
| 13 | PowerCHO-2 CD | Cell Boost 6 | 34 | 12 | 18.87 |
| 14 | CD OptiCHO | Cell Boost 6 | 34 | 15 | 15.18 |
| 15 | CD CHO 022 | CD Feed 002 | 34 | 16 | 2.82 |
| 16 | CDM4CHO | Feed A+ | 37 | 14 | 11.07 |
| 17 | CDM4CHO | Feed B+ | 37 | 12 | 8.41 |
| 18 | CDM4CHO | Feed C+ | 37 | 14 | 10.40 |
| 19 | CDM4CHO | GlycanTune C+ | 37 | 16 | 5.46 |
| 20 | HyCell CHO | Feed A+ | 37 | 14 | 24.99 |
| 21 | HyCell CHO | Feed B+ | 37 | 14 | 26.28 |
| 22 | HyCell CHO | Feed C+ | 37 | 14 | 21.40 |
| 23 | HyCell CHO | GlycanTune C+ | 37 | 15 | 19.73 |
| 24 | EX-CELL CD CHO | Feed A+ | 37 | 15 | 12.44 |
| 25 | EX-CELL CD CHO | Feed B+ | 37 | 14 | 11.37 |
| 26 | EX-CELL CD CHO | Feed C+ | 37 | 16 | 8.66 |
| 27 | EX-CELL CD CHO | GlycanTune C+ | 37 | 14 | 7.01 |
| 28 | PowerCHO-2 CD | Cell Boost 6 | 37 | 11 | 25.24 |
| 29 | CD OptiCHO | Cell Boost 6 | 37 | 12 | 12.87 |
| 30 | CD CHO 022 | CD Feed 002 | 37 | 14 | 4.46 |

As a result, as shown in Table 1, it was confirmed that the cell growth according to the temperature conditions was higher at 34 °C than at 37 °C, and it was found that the cell growth according to the medium and additive conditions was most effective under the HyCell CHO (Feed C+ or GlycanTune C+) conditions and CD OptiCHO (Cell Boost 6) conditions.

Based on these results, three culture combination candidates for HAdase PH20 production were derived:
1) CD OptiCHO + Cell Boost 6, Initial culture temperature: 34 °C
2) HyCell CHO + Feed C+, Initial culture temperature: 34 °C
3) HyCell CHO + GlycanTune C+, Initial culture temperature: 34 °C

### Example 2: Production of HAdase PH20 according to optimal medium and culture temperature change time

Based on the three culture combination candidates derived in the above Example 1, the level of HAdase PH20 production according to the culture temperature change time was confirmed as shown in the table in FIG. 1.

The initial cell inoculation concentration was 1.0 x 10⁶ cells/ml, the culture scale was 50 ml/125 ml Erlenmeyer flasks, and the cell inoculation method was to resuspend the collected cells by centrifugation at 288 x g for five minutes. The culture was performed for up to 16 days, and when the pH was lower than 6.8, 1% of a 7.5% sodium bicarbonate stock was added to correct the pH. When the glucose content was 20 mmol/l or less, 1% of a 45% D-(+)-glucose stock was added.

As a result, it was confirmed that cell growth was induced more rapidly and thus productivity was increased when the culture temperature was changed compared to the control conditions where the culture temperature was not changed.

To identify HAdase PH20, Western blotting was performed. A sample buffer was added to the sample, and the resulting mixture was boiled at 100 °C for 10 minutes, and then cooled on ice for five minutes. Electrophoresis was performed by loading the resulting product onto a 10% polyacrylamide gel and blotting was performed onto a nitrocellulose (NC) membrane at 350 mA for two hours. The membrane was blocked with 5% skim milk, treated in a refrigerator with a primary antibody diluted 1:2000, and then treated at room temperature for one hour with a secondary antibody diluted 1:5000. HAdase PH20 was identified by color development by treating with nitro blue tetrazolium/5-bromo-4-chloro-3-indolyl-phosphate (NBT/BCIP).

As a result of Western blotting to confirm the expression of HAdase PH20 according to the culture conditions, as shown in FIG. 1, it was confirmed that a large amount of truncated forms (molecular weight standard between 37 and 49 kDa) of natural human recombinant HAdase PH20 were formed under the HyCell CHO + GlycanTune C+ conditions.

Under the HyCell CHO + Feed C+ conditions, the truncated forms of PH20 tended to decrease as the culture temperature was changed to 32 °C later.

Under the CD OptiCHO medium conditions, a slight amount of truncated forms of PH20 were observed when the initial culture temperature was 37 °C, but when the initial culture temperature was 34 °C, the truncated forms of PH20 were hardly observed regardless of the culture temperature change day.

Based on the above results, the basic medium CD OptiCHO, feed medium Cell Boost 6 (15% (v/v)), and initial culture temperature of 34 °C were selected as the optimal conditions for HAdase PH20 production.

### Example 3: HAdase PH20 production according to additive combination

Based on the results of the above Example 2, as shown in the table in FIG. 2, an experiment was performed under the conditions of adding 1%, 3%, and 5% of the Cell Boost 6 feed, which was selected as the optimal additive, on day 6 of culture to the basic medium CD OptiCHO, and the conditions of adding the optimal content of 15% in two to five portions at three-day intervals.

In addition, a test was performed by adding 4 mM each of L-glutamine and GlutaMAX, and the culture solution samples were analyzed by performing Western blotting to compare the quality of the expressed proteins under each condition.

The initial cell inoculation concentration was 1.0 x 10⁶ cells/ml, and the culture scale was 50 ml/125 ml Erlenmeyer flasks, and the cell inoculation method was to resuspend the collected cells by centrifugation at 288 x g for five minutes. The culture was performed for up to 20 days, and when the pH was lower than 6.8, 1% of a 7.5% sodium bicarbonate stock was added to correct the pH. When the glucose content was 20 mmol/l or less, 1% of a 45% D-(+)-glucose stock was added.

As a result, as shown in FIG. 2, no significant difference was observed in the degree of expression of the truncated forms of PH20 according to the conditions of the addition amount and addition date of Cell Boost 6 feed, and no difference was observed in the degree of expression of the truncated forms of PH20 according to the addition of L-glutamine and GlutaMAX.

### Example 4: HAdase PH20 production according to combination of other additives

The degree of PH20 production was confirmed when peptone, an additive other than Cell Boost 6 feed selected as the optimal additive, was added.

Peptone was added at 1%, 3%, 9%, and 15% using yeast extract, soybean hydrolysate (Select Phytone), yeast hydrolysate (TC Yeastolate), and cottonseed hydrolysate (Cotton 200 UF) to produce PH20, as shown in Table 3.

The initial cell inoculation concentration was 1.0 x 10⁶ cells/ml, and the culture scale was 50 ml/125 ml Erlenmeyer flasks, and the cell inoculation method was to resuspend the collected cells by centrifugation at 288 x g for five minutes. The culture was performed for up to 20 days, and when the pH was lower than 6.8, 1% of a 7.5% sodium bicarbonate stock was added to correct the pH. When the glucose content was 20 mmol/l or less, 1% of a 45% D-(+)-glucose stock was added.

As a result, as shown in FIG. 3, regardless of the type of peptone, the overall expression level of the truncated forms of PH20 tended to be high. In particular, the expression level of the truncated forms of PH20 was found to be the highest under the conditions of adding 9% TC Yeastolate.

### Example 5: HAdase PH20 production according to additives and culture days

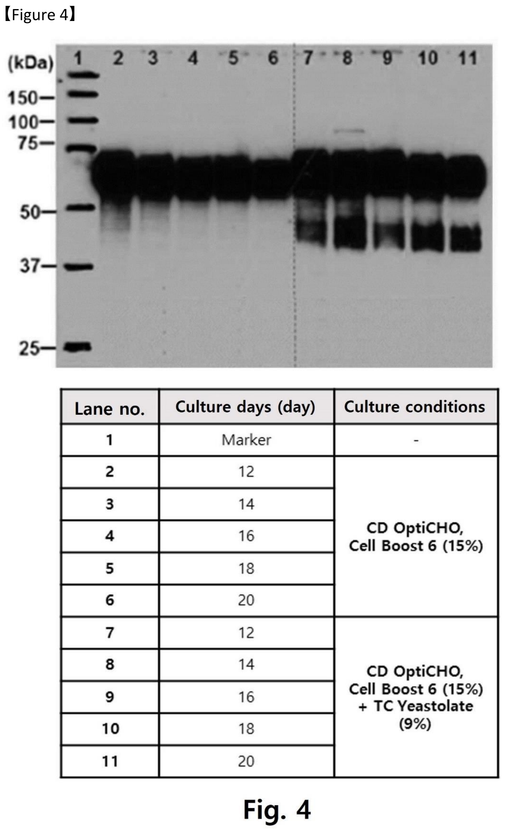

An experiment was performed in order to confirm the degree of expression of the truncated forms of PH20 by culture days under the 9% TC Yeastolate addition conditions of Example 4 and the degree of expression of the truncated forms of PH20 by culture days under the CD OptiCHO + Cell Boost 6 conditions selected in Example 3, as shown in the table in FIG. 4.

The initial cell inoculation concentration was 1.0 x 10⁶ cells/ml, and the culture scale was 50 ml/125 ml Erlenmeyer flasks, and the cell inoculation method was to resuspend the collected cells by centrifugation at 288 x g for five minutes. The culture was performed for up to 20 days, and when the pH was lower than 6.8, 1% of a 7.5% sodium bicarbonate stock was added to correct the pH. When the glucose content was 20 mmol/l or less, 1% of a 45% D-(+)-glucose stock was added.

As a result, as shown in FIG. 4, under the CD OptiCHO + Cell Boost 6 conditions, expression of the truncated forms of PH20 was not observed regardless of culture days, but under the 9% TC Yeastolate addition conditions, expression of the truncated forms of PH20 was observed on all culture days.

### Example 6: Comparison according to culture days and agitation speeds in 7.5 L bioreactor

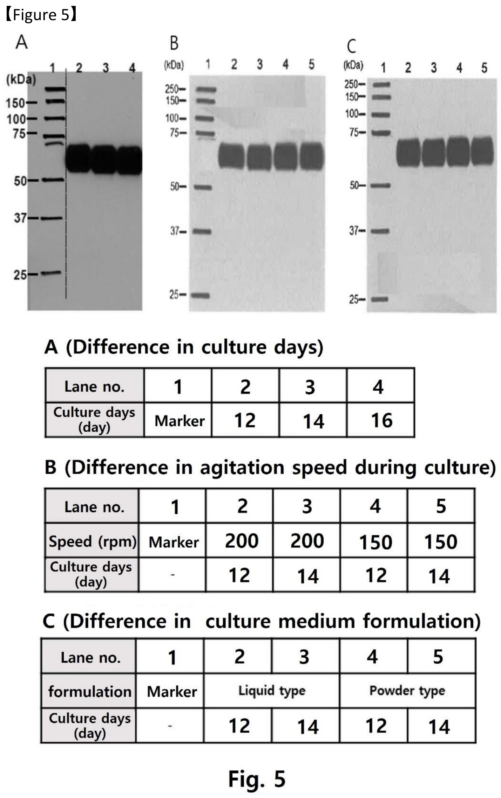

The culture was performed in a 7.5 L bioreactor by applying the culture conditions selected in the above Example 2 or Example 3, and a comparative experiment was performed according to the culture days and agitation speed (150 rpm and 200 rpm), as shown in the tables in FIGS. 5A and 5B.

The initial cell inoculation concentration was 0.3 x 10⁶ cells/ml, and the culture scale was 4 L. The cell inoculation method was to mix the seed culture sample in the required volume according to the target initial cell inoculation concentration. The culture was performed for up to 14 days, and when the pH was lower than 6.8, a 0.1 N sodium hydroxide stock was added to correct the pH until the pH reached 6.8. When the glucose content was lower than 3.5 g/l, a 45% D-(+)-glucose stock was added in an amount of 3.5 g/l of the initial culture scale. The pH range of the bioreactor was adjusted to pH 7.10 ± 0.30 on day 0 of culture, pH 7.05 ± 0.25 on day 1 of culture, and 7.00 ± 0.20 on days 2 to 14 of culture. The dissolved oxygen (DO) was maintained at 30%, and gas control was performed using a 3-gas mix.

As a result, as shown in FIG. 5A, it was confirmed that PH20 was expressed in an original form, not in its truncated forms, regardless of the culture days.

In addition, as shown in FIG. 5B, expression of the truncated forms of PH20 was not confirmed under both conditions. However, it was confirmed that when the agitation speed was 200 rpm, the DO was not stably maintained, so productivity was low, and when the agitation speed was 150 rpm, the productivity was similar to that of the existing flaks.

### Example 7: Performance comparison of liquid and powder formulations of commercial media

The selected basic medium CD OptiCHO is available in a liquid form (Thermo Fisher Scientific / Cat# 12681-011) and a powder form (Thermo Fisher Scientific / Cat# A11222-05), and an experiment was conducted to confirm the culture difference between the two forms, as shown in the table in FIG. 5C.

The initial cell inoculation concentration was 0.3 x 10⁶ cells/ml, the culture scale was 100 ml, and the cell inoculation method was to mix the seed culture sample in the required volume according to the target initial cell inoculation concentration. The culture was performed for up to 14 days, and when the pH was lower than 6.8, a 0.1 N sodium hydroxide stock was added to correct the pH until the pH reached 6.8. When the glucose content was lower than 3.5 g/l, a 45% D-(+)-glucose stock was added in an amount of 3.5 g/l of the initial culture scale.

As a result, as shown in FIG. 5C, original form PH20 was expressed regardless of the formulation of the basic medium, and no difference was observed in the final culture viable cell concentration and final productivity.

### Example 8: Purification of natural human recombinant HAdase PH20 using animal cell culture supernatant

### Step 1: First Purification by ion exchange-mixed mode chromatography

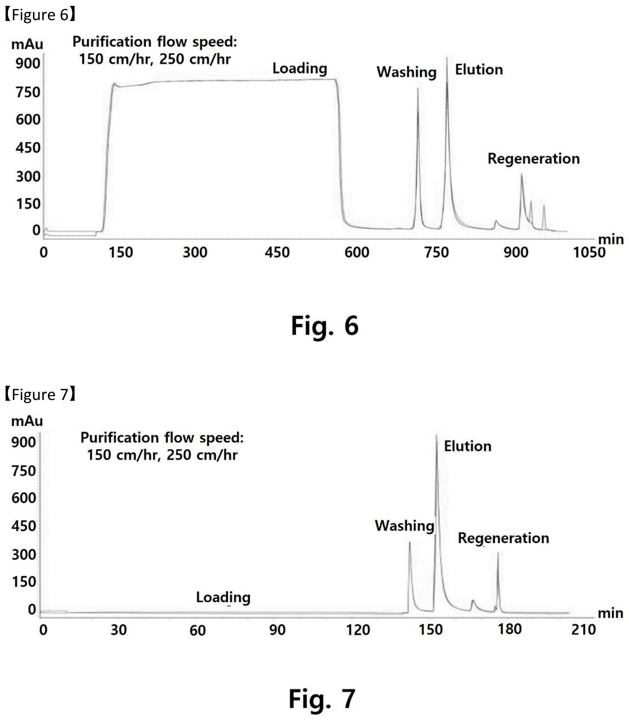

The pH of the supernatant after culture was adjusted by applying the culture conditions selected in the above Example 2 or Example 3, and then filtration was performed. The ion exchange-mixed mode column was equilibrated with a sodium acetate equilibration solution, and the filtrate was loaded onto the ion exchange-mixed mode column to remove some impurities and capture HAdase PH20. The sodium acetate equilibration solution was passed again to remove impurities that were not bound to the column, and non-specifically bound impurities were removed with a sodium phosphate washing solution. HAdase PH20 was eluted through an elution solution containing arginine in sodium phosphate, and the column was washed using a sodium hydroxide regeneration solution (FIG. 6).

### Step 2: Second purification by affinity chromatography

Water for injection was added to the eluate of Step 1 to lower the conductivity, the pH was adjusted, and filtration was performed. The affinity mode column was equilibrated with a sodium acetate equilibration solution, and the filtrate was loaded onto the affinity column to remove some impurities and allow HAdase PH20 to bind. The sodium acetate equilibration solution was passed again to remove impurities that were not bound to the column, and non-specifically bound impurities were removed with a sodium phosphate washing solution. HAdase PH20 was eluted through an eluate solution containing 250 mM sodium chloride in sodium phosphate, and the column was washed using a sodium hydroxide regeneration solution (FIG. 7).

### Step 3: Virus inactivation

Polysorbate 80 and tri-N-butyl phosphate were added to the eluate of Step 2, and the resulting mixture was allowed to stand at room temperature to inactivate the virus.

### Step 4: Third purification by ion exchange-mixed mode chromatography

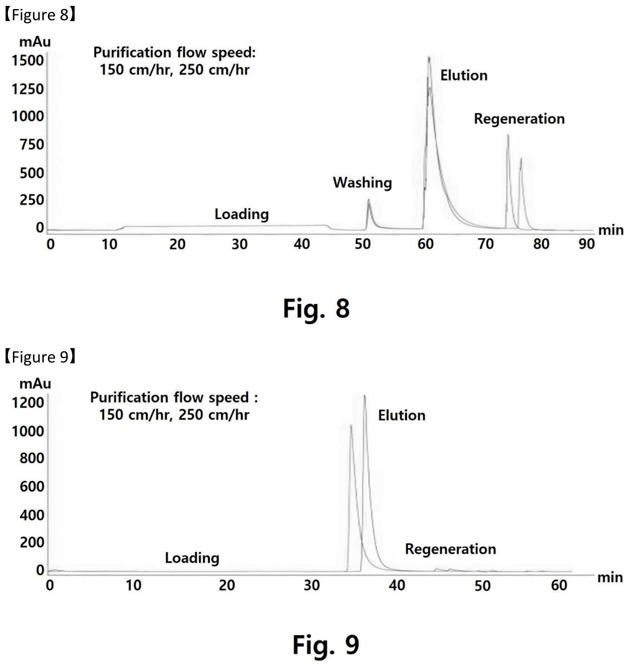

The ion exchange-mixed mode column was equilibrated with a sodium phosphate equilibration solution, and after filtrating the reaction solution of Step 3, the resulting filtrate was loaded onto the mixed mode column to remove cell-derived proteins and allow HAdase PH20 to bind. The sodium phosphate equilibration solution was passed again to remove impurities that were not bound to the column, and non-specifically bound impurities were removed with a washing solution containing 120 mM sodium chloride in sodium acetate. HAdase PH20 was eluted through an elution solution containing 750 mM sodium chloride in sodium acetate, and the column was washed using a sodium hydroxide regeneration solution (FIG. 8).

### Step 5: Fourth purification by mixed mode chromatography

A diluting solution was added to the eluate of Step 4 so that the composition became similar to that of the equilibration solution, and the resulting mixture was allowed to stand at 2 to 8 °C for 16 hours. After filtering the reaction solution, the resulting filtrate was loaded onto a mixed mode column to remove cell-derived proteins and allow HAdase PH20 to bind. The sodium phosphate equilibration solution was passed again to remove impurities that were not bound to the column, and the HAdase PH20 was eluted through a 40 mM sodium phosphate elution solution. The column was washed using a sodium hydroxide regeneration solution (FIG. 9).

### Step 6: Fifth purification by hydrophobic interaction chromatography

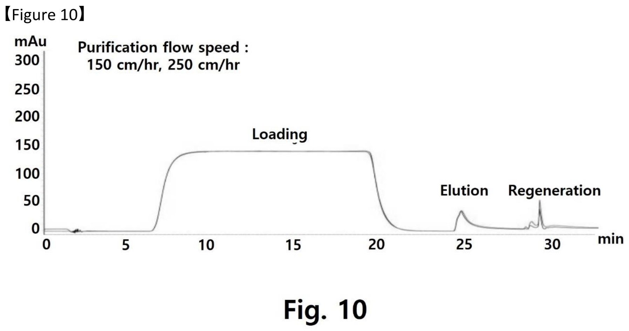

A diluting solution was added to the eluate of Step 5 so that the composition became similar to that of the equilibration solution, and after filtering the resulting mixture, the resulting filtrate was loaded onto a hydrophobic interaction column so that cell-derived DNA was bound to the column and the unbound HAdase PH20 was obtained. The sodium phosphate equilibration solution was passed again to further obtain the remaining HAdase PH20, and the column was washed using a sodium hydroxide regeneration solution (FIG. 10).

### Step 7: UF/DF and virus filtration

After Step 6, the solution was changed to a formulation solution containing sodium phosphate, sodium chloride, calcium chloride, and the like using a 30 kDa molecular weight cut-off (MWCO) membrane filter. Thereafter, the protein solution containing HAdase PH20 was filtered using a virus filter.

### Example 9: Western blot analysis of purified HAdase PH20

For a Western blot analysis of HAdase PH20, samples were prepared under two conditions, non-reducing and reducing conditions, as shown in the table in FIG. 11.

For the non-reducing conditions, 0.2, 0.5, and 1 µg samples were prepared, and then a non-reducing sample buffer was added. For the reducing conditions, a reducing sample buffer was added to 0.2, 0.5, and 1 µg samples, and the resulting mixture was boiled at 100 °C for 10 minutes, and then cooled on ice for five minutes.

Next, the sample was loaded onto a 10% polyacrylamide gel, and electrophoresis was performed under the conditions of 80 V for 30 minutes and 120 V for 100 minutes. The gel after the electrophoresis was blotted onto an NC membrane under the conditions of 250 mA for two hours. As a primary antibody, polyclonal rabbit anti-Human SPAM1 was used, and as a secondary antibody, goat anti-rabbit IgG(H+L)-AP conjugated was used

As a result, as shown in FIG. 11, original form PH20 (about 60 kDa), not the truncated forms of PH20, was confirmed under both non-reducing and reducing conditions.

### Example 10: SDS-PAGE analysis of purified HAdase PH20

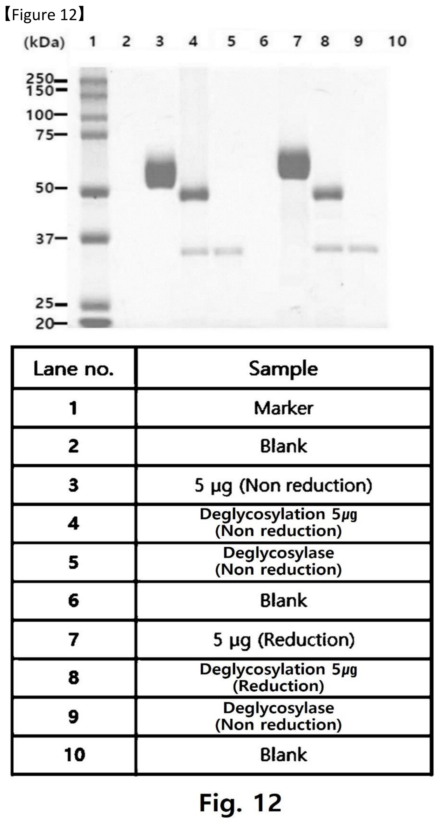

To confirm the molecular weight difference according to N-glycan deglycosylation of HAdase PH20, as shown in the table in FIG. 12, a 10x glycoprotein denaturing buffer was added, and the resulting mixture was boiled at 100 °C for 10 minutes, and NP-40 and PNGase F were added and allowed to react overnight.

Samples were prepared under two conditions, non-reducing and reducing conditions, and loaded onto a 10% polyacrylamide gel, and electrophoresis was performed under the conditions of 80 V for 30 minutes and 120 V for 100 minutes.

As a result, as shown in FIG. 12, a deglycosylated band of approximately 54 kDa was confirmed upon deglycosylation.

### Example 11: Size exclusion chromatography analysis of purified HAdase PH20

To analyze the purity of the HAdase PH20, an analysis was performed using a G2000SWXL column with a pH 7.2 solution containing sodium phosphate and sodium chloride as a mobile phase.

The Alliance high-performance liquid chromatography (HPLC) equipment from Waters was used, and the mobile phase buffer was prepared with a composition of 200 mM KPi, and 50 mM NaCl and pH 7.2, and an analytical sample was loaded in an amount of 100 µg. The flow rate was 0.5 ml/min, the column temperature was 25 °C, and the detection wavelength was 280 nm. The gradient condition was isocratic, and the analysis was performed for 40 minutes.

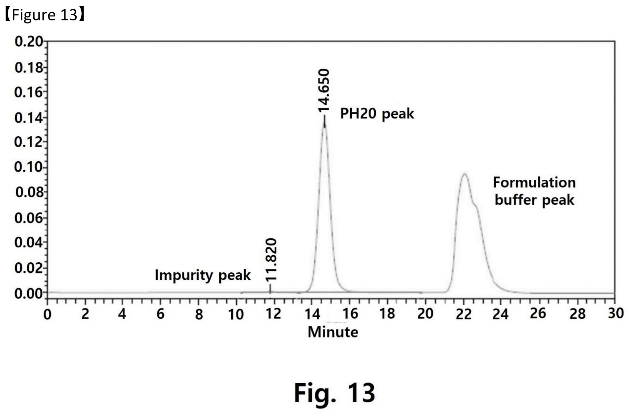

As a result, as shown in FIG. 13, it was confirmed that the purity of the original form PH20, not the truncated form of PH20, was 99%.

### [Industrial Applicability]

In the present invention, through control of the culture temperature, medium type, added feed medium components, dissolved oxygen concentration, glucose concentration, pH, and the like of host cells expressing natural human recombinant hyaluronidase (HAdase) PH20, original form HAdase PH20, not a truncated form of HAdase PH20, was produced with high purity. The original form HAdase PH20 produced by the method of the present invention has high specific activity, and thus can be effectively applied to various fields.

## Claims

1. A method for producing original form hyaluronidase PH20, comprising:
(1) culturing a host cell expressing natural human recombinant hyaluronidase PH20 at a culture temperature of 33 to 35 °C; and
(2) culturing for 7 to 11 days while maintaining the culture temperature at 31 to 33 °C,
wherein the host cell in Step (1) and/or Step (2) is cultured in a CD OptiCHO culture medium to which Cell Boost 6 feed medium is added,
and the produced hyaluronidase PH20 is present in an original form having a size of 50 to 75 kDa and not in a truncated form.

2. The method of claim 1, wherein the original form hyaluronidase PH20 has a size of 55 to 65 kDa.

3. The method of claim 1, wherein the activity of the hyaluronidase PH20 produced by the method in a culture solution is 4,000 units/ml or higher.

4. The method of claim 1, wherein the Cell Boost 6 feed medium is contained in the culture medium of the host cells in Step (1) and/or Step (2) at a concentration of 1% (v/v) to 20% (v/v).

5. The method of claim 1, wherein L-glutamine is further added to the culture medium of the host cells in Step (1) and/or Step (2) and cultured.

6. The method of claim 1, wherein no component selected from the group consisting of peptone, plant-derived hydrolysate, yeast extract, and yeast hydrolysate is added to the culture medium of the host cells in Step (1) and/or Step (2).

7. The method of claim 1, wherein the culture in Step (2) is performed by one or more methods selected from the group consisting of:
(a) culturing in which the dissolved oxygen content of a culture solution is maintained at 30% to 79%;
(b) culturing in which the residual glucose concentration in the medium is maintained at 1.11 to 5.95 g/l during culture period; and
(c) culturing in which the pH of the culture solution is maintained at 7.0 to 7.4.

8. The method of claim 1, wherein the culture of the host cells in Step (1) and/or Step (2) is performed by one or more methods selected from the group consisting of batch culture, repeated batch culture, fed-batch culture, repeated fed-batch culture, continuous culture, and perfusion culture.

9. The method of claim 1, wherein the hyaluronidase PH20 produced by the method optionally has some of the amino acid residues at an N-terminus and/or C-terminus deleted.

10. The method of claim 1, wherein the host cell is an animal cell, yeast, *Actinomycetes,* or an insect cell.

11. The method of claim 1, further comprising: (3) separating and purifying the produced hyaluronidase PH20.

12. The method of claim 11, wherein the separating and purifying in Step (3) is to purify hyaluronidase PH20 with high purity by utilizing affinity binding, ionic binding characteristics, and/or hydrophobic interaction characteristics with PH20.

13. The method of claim 11, wherein the separating and purifying is performed using affinity chromatography, ion exchange-mixed mode chromatography, and hydrophobic interaction chromatography.

14. Original form hyaluronidase PH20 having a size of 50 to 75 kDa produced by the method of any one of claims 1 to 13.
